(19) Europäisches Patentamt
European Patent Office
Office européen des brevets

(11) **EP 1 563 819 A1**

(12) **EUROPEAN PATENT APPLICATION**
published in accordance with Art. 158(3) EPC

(43) Date of publication:
**17.08.2005 Bulletin 2005/33**

(51) Int Cl.⁷: **A61J 3/00**, A61M 5/14

(21) Application number: **03772837.5**

(86) International application number:
**PCT/JP2003/014605**

(22) Date of filing: **17.11.2003**

(87) International publication number:
**WO 2004/045489 (03.06.2004 Gazette 2004/23)**

(84) Designated Contracting States:
**AT BE BG CH CY CZ DE DK EE ES FI FR GB GR HU IE IT LI LU MC NL PT RO SE SI SK TR**

(30) Priority: **18.11.2002 JP 2002334425**

(71) Applicant: **OTSUKA PHARMACEUTICAL FACTORY, INC.**
**Naruto-shi, Tokushima 772-8601 (JP)**

(72) Inventors:
• **IZUMI, Masamitsu**
**Naruto-shi, Tokushima 772-0017 (JP)**
• **IGUCHI, Seiichiro**
**Naruto-shi, Tokushima 772-0002 (JP)**
• **INOUE, Fujio**
**Naruto-shi, Tokushima 772-0041 (JP)**

(74) Representative: **Hertz, Oliver, Dr. et al**
**v. Bezold & Sozien**
**Patentanwälte**
**Akademiestrasse 7**
**80799 München (DE)**

(54) **DRIP BLENDER, MIXING TUBE, LIQUID DRUG CONTAINER, LIQUID MIXTURE CONTAINER, DRIP BLENDING SYSTEM AND METHOD OF BLENDING DRIP**

(57) It is intended to provide a systemwhich is safe to both of patients and medical staffs and by which medical accidents can be prevented. An admixture machine for infusion 1 has an information input unit for inputting predetermined information involving data concerning at least the body surface area, AUC or the body weight of the patient; a liquid delivery does calculation unit for determining the does of a liquid form drug and the amount of a diluent and calculating the delivery does of the liquid form drug and the diluent based upon the determined does; a guide unit for inserting a mixing tube which provides channels of the liquid form drug and the diluent; and liquid delivery units which deliver the liquid form drug and the diluent based on the delivery does of the liquid form drug and the diluent as determined in the liquid delivery does calculation unit.

FIG. 1

**Description**

TECHNICAL FIELD

The present invention relates to an admixture machine for infusion, a admixture preparation system, a mixing tube, a liquid form drug container, a mixing container and a admixture method of infusion, and more specifically concerns a device used for diluting an oncology drug for infusion, as well as a system, tubes, containers and a method for such a device.

BACKGROUND ART

Conventionally, most of oncology drugs for infusion work on a plurality of cycles of the cell cycle to cause cytotoxicity. Consequently, since they give adverse effects to normal cells, each anti-tumor drug is dilution-adjusted to a predetermined concentration, and then applied. In other words, the dose of each oncology drug is determined depending on the body surface area or the body weight, and the dose of liquid form drug is calculated for each patient and the corresponding does of the drug is administrated to the patient. Moreover, in the case when an administrated dose cannot be uniformly set up due to a patient's tolerance to drug dose (seriousness of adverse reaction), the administrated dose may be individually set based upon the area under the plasma drug concentration curve (AUC), and in this case, complex calculating processes are required. In addition, most of oncology drugs are included in those drugs whose carcinogenicity has been pointed out by the international cancer research institution, and the risk of handling those drugs has been pointed out. Furthermore, in some of those drugs, castor oil or the like may be used as a resolvent, and this is said that these solvents cause the adverse reaction. Most of the oncology drugs tend to have low water solubility, and also tend to cause salting out when the drug is diluted in a large amount of saline solution at once; thus, problems are raised in solubility as well as in diluent property.

For this reason, upon medication for such an anti-tumor drug for infusion, a medical staff transfers, for example, a liquid form drug of the anti-tumor drug for infusion from an ample by using syringe, and dilutes this by using a diluent suitable for the properties of the oncology drug, based upon the does of liquid form drug calculated for each of patients; thus, the drug is administrated to the patient as the corresponding mixed solution.

Moreover, conventionally, a device, which is used for filling a plurality of solutions into a single container, has been known (for example, see Patent Publication JP 2592630).

DISCLOSURE OF INVENTION

However, in the device of this type, problems have been raised when, with respect to oncology drugs to be medicated, admixture processes require calculations of the amounts of drugs for medication based upon the body surface area, body weight or AUC of a patient, and the conventional devices have failed to address these problems. Consequently, even when the admixture processes of anti-tumor drugs for infusion are carried out by using the device which fills a plurality of solutions to a single container, appropriate amounts of the liquid form drugs need to be calculated for each patient as well as for each of the liquid form drugs and diluents to be given, with respect to medication of the anti-tumor drugs, and the kinds of the liquid form drugs and the does of the liquid form drugs to be medicated need to be sufficiently managed under sterile. If an erroneous connection should be made with respect to the kinds of liquid form drugs, or if overdosage should be erroneously given due to erroneous calculations or input mistakes, or if a diluent solution should be erroneously bacterial-contaminated, there is a possibility that a patient's life might be endangered, and there is also a risk of a serious medical accident.

Moreover, medical staffs who carry out admixture processes also risk being exposed to toxic anti-tumor drugs so that it is necessary to secure the safety of those medical staffs. Most of oncology drugs are contained in glass ampules and glass vials, and since the doses of drugs are different depending on patients as described earlier, it becomes inevitably difficult to use all the content of each liquid form drug, with the result that residual solutions are left in most cases. Consequently, disposal of residual liquids and abandonment of containers pose a big problem. Thus, in medical fields in which liquid form drugs such as anti-tumor drugs for infusion need to be handled, there have been strong demands for a system which takes the environment into consideration, and is safe to both of patients and medical staffs and by which medical accidents can be prevented.

The present invention has been devised to solve the above-mentioned problems. The main objective of the present invention is to provide a admixture machine for infusion that is capable of admixing preparation predetermined dose of a liquid form drug to be administrated or given to a patient and a diluent based upon information involving data concerning the body surface area, AUC or the body weight of the patient, and a mixing tube, a liquid form drug container and a mixing container to be used in such a admixture machine for infusion, as well as a admixture preparation system and a admixture method using such a admixture machine for infusion.

In order to achieve the above-mentioned objective, a admixture machine for infusion in accordance with claim 1 of the present invention, which is used for admixture preparation predetermined does of a liquid form drug to be administrated to a patient and a diluent, is provided with: an information input unit for inputting predetermined information involving data concerning at least the body surface area, the area under the plasma drug concentration curve (AUC) or the body weight of the patient; a liquid delivery does calculation unit for determining the does of a liquid form drug and the amount of a diluent which are both to be delivered based upon the information inputted through the information input unit and calculating the delivery does of the liquid form drug and the diluent based upon the determined does thereof; a guide unit for inserting a mixing tube which provides channels of the liquid form drug and the diluent; and liquid delivery units which are in contact respectively with a liquid form drug channel through which the liquid form drug flows and a diluent channel through which the diluent flows in the mixing tube to be inserted into the guide unit and thus deliver the liquid form drug and the diluent based on the delivery does of the liquid form drug and the diluent as determined in the liquid delivery does calculation unit.

Moreover, a admixture machine for infusion in accordance with claim 2 of the present invention, which relates to the admixture machine for infusion disclosed in claim 1, is designed so that the predetermined information to be inputted to the information input unit further includes a name of a patient for medication, and this admixture machine for infusion further includes a memory unit capable of storing at least the name of the patient, the body surface area, the AUC or the body weight of the patient in association with one another, and upon input of a name of a patient into the information input unit, at least the body surface area, the AUC or the body weight in association with the inputted patient name is called for by referencing the memory unit, and automatically inputted to the information input unit. With this arrangement, by inputting a name of a patient for medication into the information input unit, predetermined does of a liquid form drug to be administrated to the patient and a dilution solution based upon information concerning the body surface area of the patient are admixed.

A admixture machine for infusion in accordance with claim 3 of the present invention, which relates to the admixture machine for infusion disclosed in claim 1 or claim 2, is designed so that the predetermined information to be inputted to the information input unit further includes the body surface area of the patient for medication, the AUC, the body weight, the name of a liquid form drug to be administrated, the does of the liquid form drug, and the does of the liquid form drug per unit body surface area or per body weight of the patient with respect to the liquid form drug or the does of the liquid form drug determined based upon the AUC, and the calculation unit automatically calculates the value of the does of the liquid form drug based upon at least the body surface area, the AUC or the body weight of the patient inputted to the information input unit, and the admixture machine for infusion further includes a memory unit that is capable of storing the name of the liquid form drug and the does of the liquid form drug per unit body surface area or per weight or the does of the liquid form drug determined based upon the AUC in association with one another and a warning unit which compares the does of the liquid form drug individually inputted from the information input unit with the value of the does of the liquid form drug automatically calculated in the calculation unit, and, when the difference is greater than a predetermined value, gives warning. With this structure, it becomes possible to prevent an excessive does of medication due to an erroneous inputting operation.

Moreover, a admixture machine for infusion in accordance with claim 4 of the present invention, which relates to the admixture machine for infusion disclosed in claim 3, is designed so that the memory unit further stores at least one piece of information selected from application and medication methods.

A admixture machine for infusion in accordance with claim 5 of the present invention, which relates to the admixture machine for infusion disclosed in claim 1 or claim 2, is designed so that based upon information given from the information input unit, the calculation unit automatically calculates the does of the liquid form drug and the amount of the dilution solution. With this arrangement, by inputting predetermined information to the information input unit, the does of the liquid form drug and the amount of the dilution solution to be supplied are automatically calculated.

Moreover, a admixture machine for infusion in accordance with claim 6 of the present invention, which relates to the admixture machine for infusion disclosed in any of claims 1 to 5, is designed so that, with respect to the inputting process of the body surface area or the AUC of the patient, by inputting parameters required for calculating the body surface area or the AUC, calculations are automatically carried out through predetermined operation expressions based upon these parameters so that the results are automatically inputted. With this arrangement, by inputting parameters required for calculating the body surface area, the admixture machine for infusion is allowed to calculate the body surface area.

A admixture machine for infusion in accordance with claim 7 of the present invention, which relates to the admixture machine for infusion disclosed in any of claims 1 to 6, is further provided with a bar-code reading unit which inputs at least one portion of pieces of information to the information input unit by reading bar codes. With this arrangement, it becomes possible to simplify the inputting operation of predetermined information to the information input unit.

Moreover, a admixture machine for infusion in accordance with claim 8 of the present invention, which relates to the admixture machine for infusion disclosed in any of claims 1 to 6, is further provided with a signal receiving unit which inputs at least one portion of pieces of information to the information input unit by using IC tag signals.

A admixture machine for infusion in accordance with claim 9 of the present invention, which relates to the admixture machine for infusion disclosed in any of claims 1 to 8, is further provided with a memory-card reading unit which inputs at least one portion of pieces of information to the information input unit by reading a memory card. With these arrangements, it becomes possible to simplify the inputting operation of predetermined information to the information input unit.

Moreover, a admixture machine for infusion in accordance with claim 10 of the present invention, which relates to the admixture machine for infusion disclosed in any of claims 1 to 9, is designed so that a printing device, which is capable of printing at least one portion of pieces of information to be inputted to the information input unit on a pasting label, is prepared in a connectable state. With this arrangement, it becomes possible to print information required for medication on a pasting label.

A admixture machine for infusion in accordance with claim 11 of the present invention, which relates to the admixture machine for infusion disclosed in any of claims 1 to 10, is further provided with a flow meter for measuring the amount of flow passing through a mixing tube to be inserted to the guide unit, and in this arrangement, the amount of flow, measured by the flowmeter, is compared with the amount of liquid delivery calculated by the calculation unit so that the quantity of liquid delivery is controlled through a feed-back controlling process. With this arrangement, it becomes possible to carry out a admixing and preparation operation with high precision.

Moreover, a admixture machine for infusion in accordance with claim 12 of the present invention, which relates to the admixture machine for infusion disclosed in any of claims 1 to 11, is designed so that the liquid form drug is an oncology drug for infusion. Thus, the admixture machine for infusion of the present invention is suitably used for admixture an anti-tumor drug for infusion.

A admixture machine for infusion in accordance with claim 13 of the present invention, which relates to the admixture machine for infusion disclosed in any of claims 1 to 12 , is designed so that the mixing tube to be inserted to the guide unit of the admixture machine for infusion is branched into a tong shape, that is, a tong structure having one of branched ends serving as a liquid form drug channel to be connected to a liquid form drug container for packing a liquid form drug, the other end serving as a diluent channel to be connected to a diluent container for packing a diluent, and the branch portion serving as a mixing channel in which the liquid form drug channel and the diluent channel are joined to each other, and in this structure, the guide unit of the admixture machine for infusion is formed into a tong shape to which the tong-shaped mixing tube is inserted. This arrangement makes it possible to utilize the mixing tube as a disposable part.

Moreover, a mixing tube in accordance with claim 14 of the present invention, which is the mixing tube to be used in the admixture machine for infusion according to any of claims 1 to 13, has a structure branched into a tong shape, that is, a tong structure having one of branched ends serving as a liquid form drug channel to be connected to a liquid form drug container for packing a liquid form drug, the other end serving as a diluent channel to be connected to a diluent container for packing a diluent, and the branch portion, in which the liquid form drug channel and the diluent channel are joined to each other, serving as a mixing channel to be connected to a mixing container for packing the mixed solution of the liquid form drug and the diluent.

Furthermore, a mixing tube in accordance with claim 15 of the present invention, which is the mixing tube to be used in the admixture machine for infusion according to any of claims 1 to 13, is provided with a liquid form drug channel one end of which is connected to a liquid form drug container for packing a liquid form drug and a diluent channel one end of which is connected to a diluent container for packing a diluent individually, with the other ends of the channels being connected to a mixing container for packing a mixed solution of the liquid form drug and the diluent.

A mixing tube in accordance with claim 16 of the present invention, which is the mixing tube to be used in the admixture machine for infusion according to claim 14 or claim 15, is designed so that the mixing tube is provided with a liquid form drug container connecting port to be connected to a port of the liquid form drug container at an end of the liquid form drug channel and a diluent container connecting port to be connected to a port of the diluent container at an end of the diluent channel, with the liquid form drug container connecting port being formed into a shape that is only connectable to the liquid form drug container. With this arrangement, it becomes possible to prevent an erroneous connection of the liquid form drug container.

A mixing tube in accordance with claim 17 of the present invention, which is the mixing tube to be used in the admixture machine for infusion according to any of claims 14 to 16, is designed so that the liquid form drug container connecting port of the mixing tube has a securing member used for securing the port of the liquid form drug container. This arrangement makes it possible to positively secure the liquid form drug container connecting port and the port of the liquid form drug container.

A liquid form drug container in accordance with claim 18 of the present invention, which is the liquid form drug container to be used in the admixture machine for infusion according to any of claims 1 to 13, is provided with: a discharging outlet, placed at its part, which is connected to a liquid form drug container connecting port formed at an end portion of the liquid form drug channel.

Moreover, a liquid form drug container in accordance with claim 19 of the present invention, which is the liquid form drug container according to claim 18, is designed so that liquid form drugs that have been primarily diluted are contained in the liquid form drug container in a plurality of quantity to be applied. With this arrangement, admixing and preparation operations can be carried out for a plurality of patients without the necessity of exchanging the liquid form drug containers.

A liquid form drug container in accordance with claim 20 of the present invention, which is the liquid form drug container according to claim 18, is designed so that undiluted liquid form drugs are contained in the liquid form drug container in a plurality of quantity to be applied.

Moreover, a liquid form drug container in accordance with claim 21 of the present invention, which is the liquid form drug container according to claim 18, is provided with a liquid form drug packing unit for packing the liquid form drug, and a primary diluent packing unit for packing a primary diluent that is used for primarily diluting the liquid form drug, and in this structure, a communicating unit, which is used for mixing the liquid form drug and the primary diluent, is placed between the liquid form drug packing unit and the primary diluent packing unit. With this arrangement, it becomes possible to carry out a admixture process with high precision. Moreover, since a liquid form drug, which has been already primary-diluted, is connected to the mixing tube, it becomes possible to lessen the risk to medical staffs upon connection.

A liquid form drug container in accordance with claim 22 of the present invention, which is the liquid form drug container according to claim 21, is prepared as a plastic bag having two chambers separated by an easily-peelable seal, with one of the chambers packing an undiluted liquid form drug and the other chamber packing a primary diluent.

Moreover, a liquid form drug container in accordance with claim 23 of the present invention, which is the liquid form drug container according to claim 18, is provided with a connecting unit, placed at a position virtually opposing to the port of the liquid form drug container, which allows transfer with another liquid form drug container. This arrangement allows a primary diluent or a higher-order diluent.

A liquid form drug container in accordance with claim 24 of the present invention, which relates to the liquid form drug container according to claim 23, is designed so that the connecting unit is provided with a double-sided needle or transfer needle. This arrangement makes it possible to easily break the connecting portion between the respective containers to positively allow transfer between the containers.

A mixing container in accordance with claim 25 of the present invention, which is used in the admixture machine for infusion according to any of claims 1 to 13, is designed so that the mixing container is integrally formed with the mixing tube.

A admixture preparation system of infusion in accordance with claim 26 of the present invention, which has the admixture machine for infusion and the mixing tube according to any of claims 1 to 13, is designed so that the mixing tube to be inserted to the guide unit of the admixture machine for infusion has a structure branched into a tong shape, that is, a tong structure having one of branched ends serving as a liquid form drug channel to be connected to a liquid form drug container for packing a liquid form drug, the other end serving as a diluent channel to be connected to a diluent container for packing a diluent, and the branch portion, in which the liquid form drug channel and the diluent channel are joined to each other, serving as a mixing channel to be connected to a mixing container for packing the mixed solution of the liquid form drug and the diluent; and in this structure, the guide unit of the admixture machine for infusion has at least a liquid form drug channel insertion unit to which the liquid form drug channel is inserted and a diluent channel insertion unit to which the diluent channel is inserted.

Moreover, a admixture preparation system of infusion in accordance with claim 27 of the present invention, which has the admixture machine for infusion and the mixing tube according to any of claims 1 to 13, is designed so that the mixing tube to be inserted to the guide unit of the admixture machine for infusion has a liquid form drug channel that is connected to a liquid form drug container for packing a liquid form drug and a diluent channel that is connected to a diluent container for packing a diluent, with one end of the liquid form drug channel being connected to the liquid form drug container and one end of the diluent channel being connected to the diluent container; and in this structure, the guide unit of the admixture machine for infusion has at least a liquid form drug channel insertion unit to which the liquid form drug channel is inserted and a diluent channel insertion unit to which the diluent channel is inserted.

Furthermore, a admixture preparation system of infusion in accordance with claim 28 of the present invention, which has the admixture machine for infusion and the mixing tube according to claim 26 or claim 27, is designed so that the mixing tube to be inserted to the guide unit of the admixture machine for infusion is integrally formed with the admixture machine for infusion.

A admixture preparation system of infusion in accordance with claim 29 of the present invention, which relates to the admixture preparation system according to claim 26 or claim 27, is further provided with a container weight-measuring unit which allows controls on the does of the liquid form drug and the diluent by feeding back weight changes in the diluent container or the mixing container.

A admixture method of infusion in accordance with claim 30 of the present invention, in which a admixture machine for infusion for admixture preparation predetermined quantity of a liquid form drug to be administrated to a patient and a diluent is used, is provided with the steps of: inputting predetermined information involving data concerning at least the body surface area, the AUC or the body weight of the patient to the admixture machine for infusion; allowing the admixture machine for infusion to determine the does of a liquid form drug and the amount of a diluent which are both to be delivered based upon the inputted information, and also to calculate the delivery does of the liquid form drug and the diluent based upon the determined quantity thereof; and based on the delivery does of the liquid form drug and the diluent that have been calculated, allowing liquid delivery units, formed at a guide unit for inserting a mixing tube which provides channels of the liquid form drug and the diluent to the admixture machine for infusion, to respectively contact with the liquid form drug channel forming a channel for the liquid form drug and the diluent channel forming a channel for the diluent in the mixing tube so that the liquid form drug and the diluent are respectively delivered.

In accordance with a admixture machine for infusion, a mixing tube, a liquid form drug container, a mixing container, a admixture preparation system and a admixture method of infusion, a liquid form drug such as an anti-tumor drug for infusion can be appropriately administrated to a patient, and medical accidents such as an erroneous setting of the does of medication and an erroneous selection of the kind of a liquid form drug can be prevented; moreover, with respect to medical staffs, it is possible to eliminate the risk of being exposed to a cytotoxic agent, and consequently to improve the safety.

BRIEF DESCRIPTION OF DRAWINGS

Fig. 1 is an outside drawing that shows an admixture machine for infusion in accordance with one embodiment of the present invention.

Fig. 2 is a block diagram that shows an admixture preparation system in accordance with the embodiment of the present invention.

Fig. 3 is a block diagram that shows one example of a calculation unit in accordance with the embodiment of the present invention.

Fig. 4 is a block diagram that shows an admixture preparation system in accordance with another embodiment of the present invention.

Fig. 5 is a schematic diagram that shows a mixing tube in accordance with the embodiment of the present invention.

Fig. 6 is a schematic diagram that shows a liquid form drug container in accordance with the embodiment of the present invention.

Fig. 7 is an outside drawing that shows one example of a liquid form drug container connecting port of a mixing tube and a port of the liquid form drug container in accordance with the embodiment of the present invention.

Fig. 8 is an outside drawing that shows an admixture preparation system in accordance with the embodiment of the present invention in which the mixing tube is attached to the admixture machine for infusion.

Fig. 9 is an outside drawing that shows an admixture preparation system in accordance with another embodiment of the present invention in which the mixing tube is attached to the admixture machine for infusion.

Fig. 10 is a schematic diagram that shows an example in which a cover part is attached to the tip of the mixing tube in the embodiment in which the mixing container and the mixing tube are not integrally formed.

Fig. 11 is a schematic diagram that shows a state in which the mixing container is removed from the mixing tube in the embodiment in which the mixing container and the mixing tube are integrally formed.

BEST MODE FOR CARRYING OUT THE INVENTION

Referring to the drawings, the following description will discuss embodiments of the present invention. The following embodiments only exemplify a admixture machine for infusion, a mixing tube, a liquid form drug container, a mixing container, a admixture preparation system and a admixture method of infusion so as to realize the technical idea of the present invention, and the present embodiments are not intended to limit the admixture machine for infusion, mixing tube, liquid form drug container, mixing container, admixture preparation system and admixture method of infusion of the present invention to those described below. Moreover, those parts disclosed in the claims are not intended to be limited by those described in the following embodiments. Here, the size, the positional relationship and the like of the parts shown in the respective drawings are sometimes exaggerated for convenience of explanation. Moreover, with respect to the components forming the present invention, a plurality of components may be constituted by the same part so that one part may compatibly serve as a plurality of components.

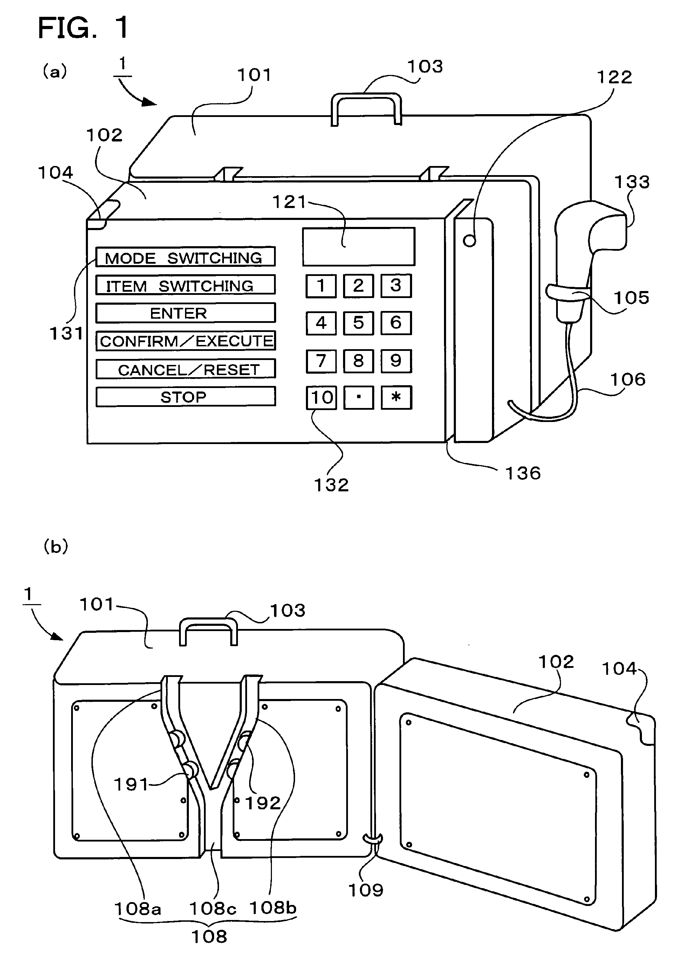

Referring to Figs. 1 to 11, the following description will discuss embodiments of the present invention in succession.

(Admixture machine for infusion)

Fig. 1 is an outside drawing that shows an admixture machine 1 for drip infusion accordance with one embodiment of the present invention. Fig. 1(a) is an outside drawing that shows a state in which a door portion 102 is closed, and Fig. 1(b) is an outside drawing that shows a state in which the door portion 102 is open. This admixture machine 1 is constituted by a main body 101 and the door portion 102. The main body 101 is provided with a handle 103 used for handling and carrying the admixture machine 1. An open-close button 104 that is used for opening and closing the door portion 102, input keys 131, ten keys 132, a display device 121, a power supply lamp 122 and a memory card reader (memory-card reading unit) 136 are placed on the door portion 102. The admixture machine 1 is further provided with a bar-code reader 133 serving as a bar-code reading unit, which is connected to a bar-code reader connecting cable 106 that is drawn from the door portion 102. The bar-code reader 133 can be attached to the main body 101 by using a bar-code reader attaching unit 105 placed on a side face of the main body 101. The input keys 131 include, for example, "a mode-switching key" used for switching operation modes of the admixture machine 1, "an item-switching key" used for switching input items in the respective operation modes, "an enter key" used for confirming the inputted item, " a confirmation/execution key" used for carrying out a confirming process on the inputted item or executing an operation of the admixture machine 1, "a cancel/reset key" used for canceling the inputted item or resetting the operation mode of the admixture machine 1 and "a stop key" used for stopping the operation of the admixture machine 1.

In the admixture machine 1 shown in Fig. 1, numeric-value inputting processes are carried out through the ten keys 132. For convenience of explanation, an input key 131 is placed for each of input items; however, it is not necessary to install a plurality of input keys, and one or two or more input keys may of course be shared with a plurality of input items. Moreover, with respect to the inputting processes such as numeric-value inputting processes, not limited to the ten keys, other input means such as a key board and a touch screen may be utilized. Moreover, with respect to the memory card, a card one portion of which is provided with a magnetic tape, a semiconductor memory or the like may be utilized.

As shown in Fig. 1(b), a guide unit 108, which is formed into a tong shape, is placed on the front face of the main body 101 that is seen when the door portion 102 is opened. This guide unit 108 is constituted by a liquid form drug channel guide 108a that forms a liquid form drug channel insertion unit in which a liquid form drug channel is inserted, a diluent channel guide 108b that forms a diluent channel insertion unit in which a diluent channel is inserted and a mixing channel guide 108c that forms a mixing channel insertion unit in which a mixing channel is inserted. Here, a liquid form drug delivery unit 191 and a diluent delivery unit 192, which serve as liquid delivery units, are respectively installed in the liquid form drug channel guide 108a and the diluent channel guide 108b. These liquid form drug delivery unit 191 and diluent delivery unit 192 are constituted by, for example, rollers. The input keys 131, the ten keys 132, the display device 121, the power-supply lamp 122, the memory-card reader 136 and the bar-code reader 133 that is connected to the bar-code reader connecting cable 106 drawn from the door portion 102, which are placed on the door portion 102, are electrically connected to the main body 101 through door-portion connecting cables 109. Here, the ten keys or the entire door portion may be prepared as detachable parts so as to carry out radio inputting operations.

(Admixture preparation system)

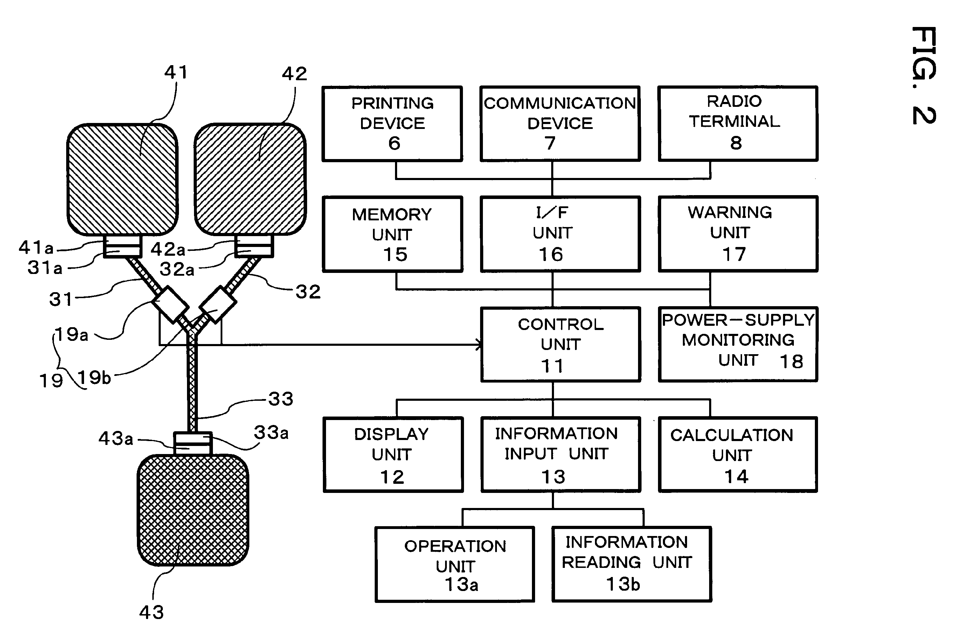

Fig. 2 is a block diagram that shows an admixture preparation system in accordance with the embodiment of the present invention. As shown in Fig. 1, the admixture machine 1 is provided with an operation unit 13a constituted by the input keys 131, the ten keys 132, etc., and an information reading unit 13b constituted by the bar-code reader 133, the memory-card reader 136, etc. The admixture preparation system includes an information input unit 13 used for inputting predetermined information concerning at least any one of items including the body surface area, the AUC and the body weight of a patient, through these operation unit 13a and information reading unit 13b; a liquid delivery does calculation unit 14 for determining the does of a liquid form drug and the amount of a diluent which are both to be delivered based upon the information inputted through the information input unit 13, and for calculating the delivery does of the liquid form drug and the diluent per unit time based upon the amounts; a display unit 12 constituted by a display device 121 for displaying information and guidance inputted through the information input unit 13, the operation state and the like, a power-supply lamp 122 and the like; a memory unit 15 which can store at least any one of items including the body surface area, the AUC and the body weight of a patient in association with the patient name, and also store a control program and the like of the admixture machine 1; a liquid delivery unit 19 constituted by a liquid form drug delivery unit 19a and a diluent delivery unit 19b which respectively have a liquid form drug delivery unit 191 and a diluent delivery unit 192 that are made in contact with a liquid form drug channel 31 and a diluent channel 32 of a mixing tube 3 to be inserted to the guide unit based upon the respective does of liquid delivery of the liquid form drug and the diluent calculated in the calculation unit 14 so as to deliver the liquid form drug and the diluent; a warning unit 17 constituted by an alarm and the like; a power-supply monitoring unit 18 for detecting interception of a current supply, such as a power failure and a power supply plug disconnection; and a control unit 11 for controlling the admixture machine 1 based upon controlling programs. The admixture machine 1 is further provided with a printing device 6 that is capable of printing at least one portion of pieces of information inputted to the information input unit 13 on a pasting label that is attached to, for example, a bag-shaped mixing container, a communication device 7 through which at least one portion of pieces of predetermined information to be inputted to the information input unit 13 can be inputted through an online input or the like from an ordering system, an electronic medical report system or the like, and an interface unit 16 which can be connected to radio terminals 8 such as PHS' s so as to carry out a writing operation in an IC tag through communication with the IC tag.

The predetermined pieces of information to be inputted to the information input unit 13 include, for example, the body surface area of a patient for medication, the name of a liquid form drug to be medicated, the does of the liquid form drug and the does of the liquid form drug per unit body surface area of the patient with respect to the liquid form drug. The memory unit 15 may be designed to store the name of a liquid form drug and the does of the liquid form drug per unit body surface area of the patient with respect to the liquid form drug in association with each other. In addition, the application and the medication method may be stored in association with one another. Moreover, the names of diluents that are not applicable together with a specific liquid form drug may be stored in association with the data. Furthermore, only the names of diluents that are used for a specific liquid form drug may be stored in association with the data. If necessary, the liquid withdrawing speed of a diluent may be stored in association with the data. With respect to the predetermined pieces of information to be inputted to the information input unit 13, data, received from an ordering system, an electronic medical report system or the like through the communication device 7, may be inputted through an online input or the like via the interface unit 16.

Next, with respect to the control unit 11, the following description will discuss an example concerning the body surface area of a patient. Here, in place of the body surface area, the following example may of course be utilized based upon the other item, such as the body weight and the AUC or the combination thereof. Upon input of a patient name and a liquid form drug name into the information input unit 13, the control unit 11 reads the body surface area and the does of a liquid form drug per unit body surface area of the liquid form drug associated with the inputted patient name from the memory unit 15, and automatically inputs the body surface area and the does of a liquid form drug per unit body surface area of the liquid form drug associated with the inputted patient name to the information input unit 13. The calculation unit 14 is designed to automatically find the calculated value of the does of the liquid form drug based upon the body surface area of the patient and the does of the liquid form drug to be medicated per unit body surface area of the liquid form drug that have been inputted to the information input unit 13. The does of the liquid form drug V that is applied to the patient for medication is obtained by the following expression: (body surface area S of a patient for medication) $\times$ (does of liquid form drug K per unit body surface area). The control unit 11 may have a structure in which the does of the liquid form drug individually inputted through the information input unit 13 and the does of the liquid form drug V applicable to the patient for medication, automatically calculated by the calculation unit 14, are compared with each other, and when the difference is greater than a predetermined value, that is, for example, an upper limit does, the warning unit 17 is allowed to discharge warning.

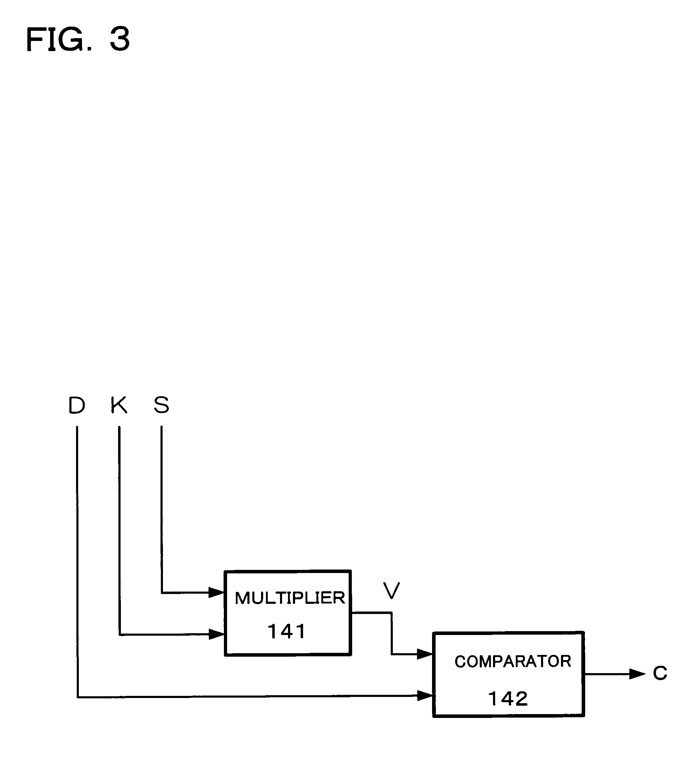

Fig. 3 shows one example of the calculation unit 14 in accordance with the embodiment of the present invention. The calculation unit 14 is provided with a multiplier 141 and a comparator 142. The control unit 11 inputs the body surface area S and the does of a liquid form drug K to be applied per unit body surface area of the liquid form drug, associated with the patient name for medication, that have been read from the memory unit 15 into the information input unit 13 that is formed by, for example, a high-speed reading memory. The body surface area S and the does of a liquid form drug K to be applied per unit body surface area of the liquid form drug, associated with the patient name for medication, that have been inputted to the information input unit 13 are respectively inputted to the multiplier 141 of the calculation unit 14 so that the standard does of the liquid form drug V to be applied to the patient for medication is calculated. Moreover, the standard does of the liquid form drug V to be applied to the patient for medication calculated in the multiplier 141 and an upper limit does D preliminarily supplied from the information input unit 13 are inputted to the comparator 142. The comparator 142 compares the upper limit does D preliminarily supplied from the information input unit 13 with the does of the liquid form drug V applicable to the patient for medication, automatically calculated by the calculation unit 14, and outputs the result of determination as to whether or not the difference is greater than the predetermined value to the control unit 11. Based upon the result of determination outputted from the calculation unit 14, the control unit 11 compares the upper limit does D preliminarily supplied from the information input unit 13 with the does of the liquid form drug V applicable to the patient for medication, automatically calculated by the calculation unit 14, and determines whether or not the difference is greater than the predetermined value, and controls the warning unit 17 to give warning based upon the result. The predetermined value is not limited to a fixed value, and may be designed so that, when the upper limit does D preliminarily supplied from the information input unit 13 is greater than the does of the liquid form drug V applicable to the patient for medication, automatically calculated by the calculation unit 14, by one digit or more, warning is given from the warning unit 17. Here, the information input unit 13 may be formed as an integral part with the control unit 11 serving as a CPU with built-in memory.

This embodiment has exemplified an arrangement in which the control unit 11 compares the upper limit does D preliminarily supplied from the information input unit 13 with the does of the liquid form drug V applicable to the patient for medication, automatically calculated by the calculation unit 14, to determine whether or not the difference is greater than a predetermined value and controls the warning unit 17 to give warning, if necessary; however, another arrangement may be used in which: by inputting the name of a patient for medication and the name of a liquid form drug to be medicated into the information input unit 13, the does of the liquid form drug V applicable to the patient for medication and an amount of a diluent required for the liquid form drug are calculated and based upon the result of calculations, the liquid form drug delivery unit 19a and the diluent delivery unit 19b are driven and controlled. Moreover, not limited to a hardware structure, the calculation unit 14 may be formed on a software basis.

With respect to the body surface area of a patient, the result of calculations preliminarily carried out may be inputted and stored in the memory unit 15, or parameters required for calculating the body surface area are inputted so that, based upon these parameters, the calculation unit 14 may automatically calculate the body surface area of the patient through predetermined operation expressions. The calculations of the body surface area may be carried out based upon, for example, DuBois's formula. Formula of DuBois approximately calculates the body surface area through the following expression 1 based upon the body weight and height.

Expression 1

$$\text{Body surface area } [m^2] = (\text{body weight } [kg])^{0.425} \times (\text{height } [cm])^{0.725} \times 0.007184$$

In addition to the above-mentioned method, the body surface area may be calculated by using another known method.

Moreover, the warning unit 17 may be designed so that, when the power-supply monitoring unit 18 detects an interception of a power supply, such as a power failure, a power supply plug disconnection and a battery shortage, it gives warning based upon the detection of the interception of a power supply.

Furthermore, for example, a mixing tube insertion sensor may be placed in each of the liquid form drug delivery unit 19a and the diluent delivery unit 19b, and when the insertion of the mixing tube 3 into the guide unit 108 is insufficient, the warning unit 17 is allowed to give warning. The mixing tube insertion sensor may be placed in the vicinity of the delivery unit of the guide unit 108, and designed so that, upon contact with the mixing tube 3, it is allowed to detect the insertion of the mixing tube 3 into the guide unit 108, or may be allowed to detect the driving resistance of the delivery unit, that is, for example, the total count the driven delivery unit (total count of rotation, etc.) in response to a driving signal inputted to the delivery unit, and designed so that, by comparing the driving resistance with a predetermined value, it is allowed to detect the insertion of the mixing tube 3 into the guide unit 108.

The warning unit 17 may be prepared as an audible warning means such as a speaker that outputs an alarm like a warning sound or gives voice warning, or may be prepared as a visual warning means by compatibly using the display unit 12, or may use these means in combination.

The liquid form drug delivery unit 19a and the diluent delivery unit 19b may respectively have flowmeters. For example, each flow meter may have a structure that electrically measures the flow rate based upon the degree of ionization of ions in the liquid form drug and diluent. In the case when the ion measuring flow meter is used, the degrees of ionization of ions in the respective liquid form drugs and diluents are preliminarily stored, and based upon the amount of ions that have passed and the degree of ionization of the ions, the flow rate can be measured. The flow rates of the liquid form drug and the diluent that have been measured by the flow meters are fed back to the control unit 11, and based upon the measured flow rates of the liquid form drug and the diluent, the control unit 11 feed-back controls driving operations of the liquid form drug delivery unit 19a and the diluent delivery unit 19b. With respect to these feed-back controlling operations, when the total flow rates of the does of the liquid form drug and the amount of the diluent, measured by the flow meters, have reached the does of the liquid form drug and the amount of the diluent to be supplied, the driving operations of the liquid form drug delivery unit 19a and the diluent delivery unit 19b may be controlled to stop, or by preliminarily calculating the does of the liquid form drug delivery and the amount of the diluent delivery per unit time using the calculation unit 14, the does of the liquid form drug delivery and the amount of the diluent delivery per unit time, measured by the flow meters, may be controlled to correspond to the does of the liquid form drug delivery and the amount of the diluent delivery per unit time that have been calculated. With this arrangement, it becomes possible to carry out an admixture process with higher accuracy.

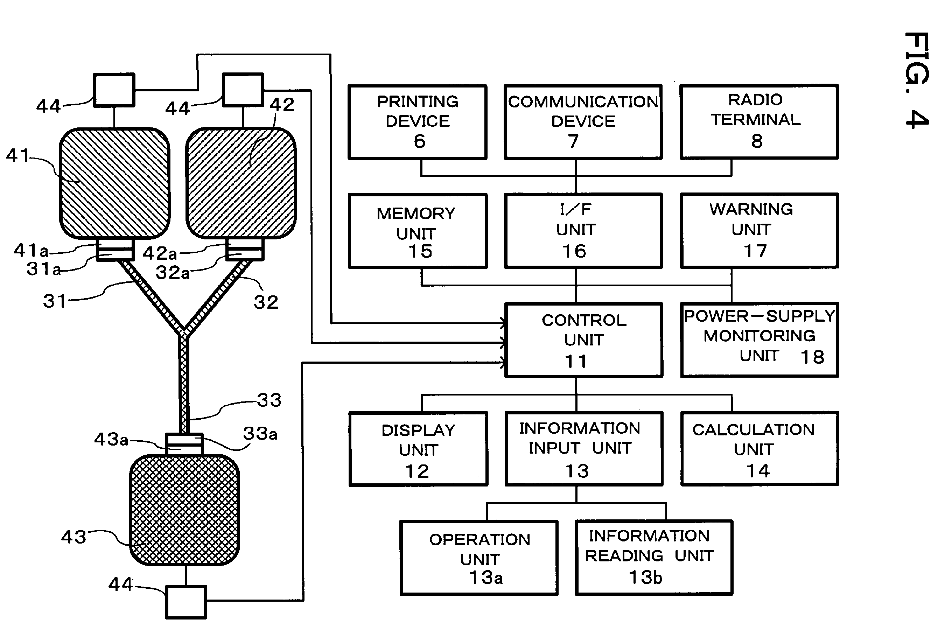

Here, the does of the liquid form drug delivery and the amount of the diluent delivery may be controlled based upon mass. In this case, in place of the flow meters, or in addition to the flow meters, for example, a container weight measuring unit is attached as means for measuring the weight differences in the diluent container and the mixing container. In addition to a structure in which it is combined into the admixture machine 1, as shown in Fig. 4, the container weight measuring unit may be connected to the admixture machine 1 as a part separated from the admixture machine 1. The container weight measuring unit 44, shown in Fig. 4, is arranged so that weight sensors are respectively attached to a liquid form drug container 41, a diluent container 42 and a mixing container 43, while data concerning the changes in each weight of container are transmitted to the control unit 11. It is not necessary to install the weight sensors in all the containers, and the weight sensors may be installed, for example, only in the liquid form drug container 41 and the diluent container 42.

The printing device 6 prints at least one portion of pieces of information inputted to the information input unit 13 on a pasting label that is attached to the mixing container 43. By attaching this pasting label to the mixing container 43, the mixing container 43 is allowed to have information required for medication. To the admixture machine of the present invention, the printing device 6, which is capable of printing at least one portion of pieces of information to be inputted to the information input unit 13 on a pasting label, can be connected through, for example, the interface unit 16. Theprintingitems, prepared by the printing device 6, may include character items, such as the name of a patient for medication, the body surface area of the patient for medication, a liquid form drug name, the does of the liquid form drug, a diluent name, the amount of the diluent, the date and time of admixture and the person in charge of the admixture, and these pieces of information may be printed as various forms such as barcodes.

Moreover, an IC tag may be pasted to the mixing container 43 so that the mixing container 43 is allowed to have information required for medication. A radio terminal 8, such as PHS, carries out a writing process of at least one portion of pieces of predetermined information to be inputted to the information input unit 13 on the IC tag by communicating with the IC tag attached to the mixing container 43. When a communication device 7, which communicates with another device, is capable of communicating with the IC tag, the writing process to the IC tag may be carried out in a compatible manner with the communication device 7. Here, the printing device 6, the communication device 7 and the radio terminal 8 may be integrally formed in the admixture machine 1.

(Mixing tube)

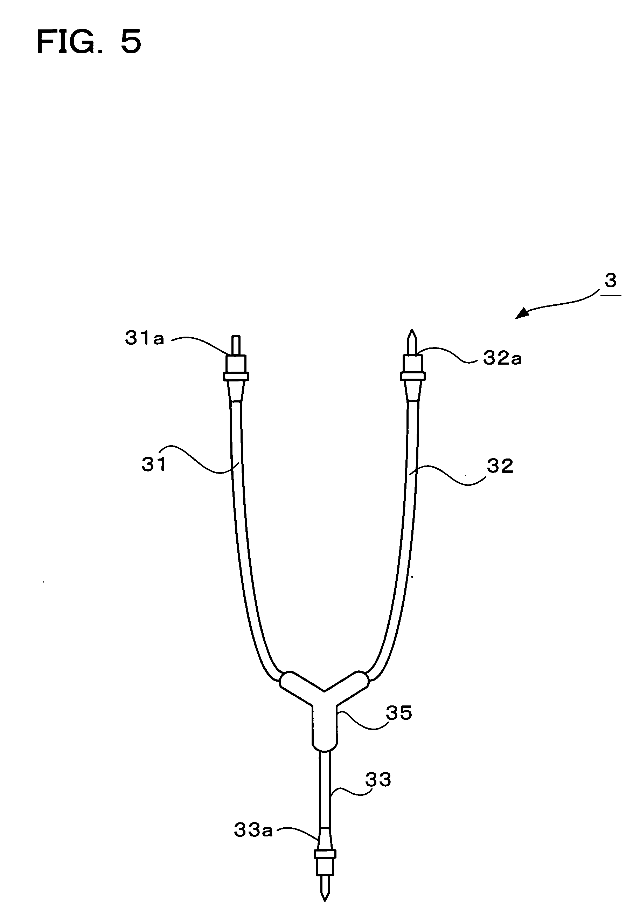

Fig. 5 is a drawing that schematically shows a mixing tube 3 in accordance with the embodiment of the present invention. This mixing tube 3 is branched into a tong shape, and the tong shape is designed so that one of the branched ends forms the liquid form drug channel 31 that is connected to the liquid form drug container 41 for packing a liquid form drug and the other end forms the diluent channel 32 that is connected to the diluent container 42 for packing a diluent, with the branched portion in which the liquid form drug channel and the diluent channel are joined to each other being prepared as the mixing channel 33. A connector 35 is placed at a joining portion among the liquid form drug channel 31, the diluent channel 32 and the mixing channel 33. The mixing container 43 for packing a mixed solution is connected to the tip of the mixing channel 33. The connections between the liquid form drug container 41 and the liquid form drug delivery unit 31, between the diluent container 42 and the diluent channel 32 as well as between the mixing channel 33 and the mixing container 43 are made by connecting a liquid form drug container port portion 41a installed in the liquid form drug container 41 to a liquid form drug container connecting port 31a attached to the tip of the liquid form drug channel 31, by connecting a diluent container port portion 42a installed in the diluent container 42 to a diluent container connecting port 32a attached to the tip of the diluent channel 32, as well as by connecting a mixing container port portion 43a installed in the mixing container 43 to a mixing container connecting port 33a attached to the tip of the mixing channel 33. Here, if necessary, a known reverse-flow stopping means, such as a check valve cock may be placed in the connector 35.

The liquid form drug container connecting port 31a is preferably formed into a shape that can be connected only to the port portion 41a of the liquid form drug container 41. This structure makes it possible to prevent an erroneous connection between the liquid form drug container 41 and the diluent container 42, or an erroneous connection to another container or another set for infusion. The mixing tube 3 is made from, for example, polyolef in such as polybutadiene or non-PVC plastics such as silicone resin. A membrane filter may be inserted into a channel, if necessary.

(Liquid form drug container)

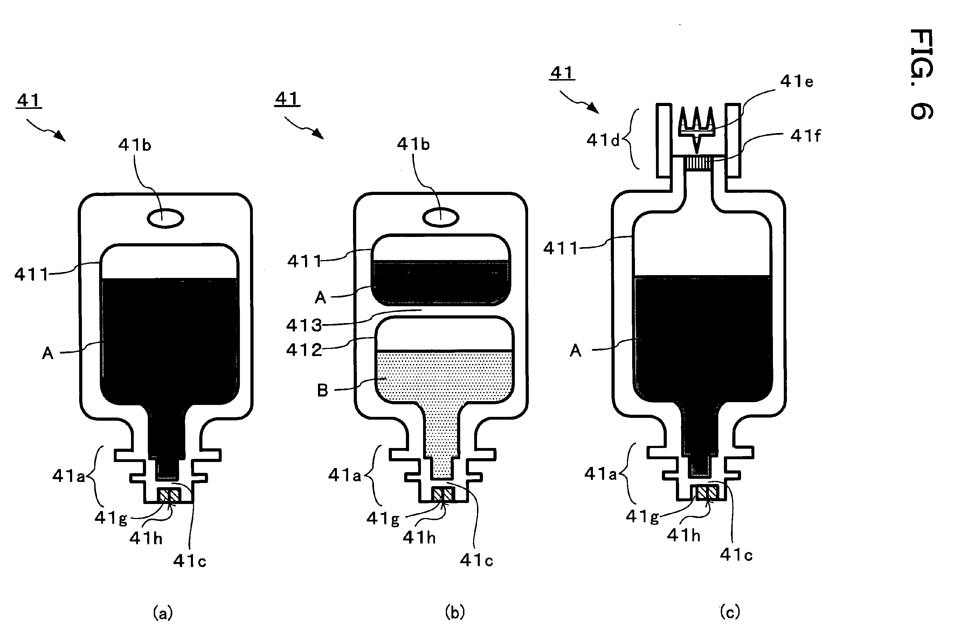

Fig. 6 is a drawing that schematically shows a liquid form drug container 41 in accordance with the embodiment of the present invention. The liquid form drug container 41 of Fig. 6(a) is prepared as a plastic bag that is provided with a liquid form drug packing unit 411 filled with an anti-tumor drug for infusion serving as a liquid form drug A, a suspending-device insertion hole 41b used for suspending the liquid form drug container 41 onto a suspending device such as a hook, a port portion 41a of the liquid form drug container 41 which is allowed to attach to the liquid form drug packing unit 411 placed at a position opposite to the position at which the suspending-device insertion hole 41b is placed, that is, a position virtually facing the suspending-device insertion unit 41b, and connected to a liquid form drug container connecting port 31a of the mixing tube 3. The port portion 41a of the liquid form drug container 41 is provided with a liquid form drug closing portion 41c that seals the liquid form drug packing unit 411. This liquid form drug closing portion 41c is designed in such a manner, when connected to the liquid form drug container connecting port 31a, the liquid form drug closing portion 41c is ruptured so as to allow the liquid form drug channel 31 of the mixing tube 3 to attach to the liquid form drug packing unit 411 of the liquid form drug container 41. An elastic sealing member 41g, for example, a rubber stopper, may be used as the liquid form drug closing portion 41c. With respect to the elastic sealing member 41g, a structure that makes the liquid form drug container connecting port 31a easily pierced, that is, for example, a notch 41h, may be formed therein. Moreover, the entire portion or one portion of the liquid form drug container 41 may be covered with a peeling film or the like.

The port portion 41a of the liquid form drug container 41 is preferably formed into a shape that can be connected only to the liquid form drug container connecting port 31a. With respect to the anti-tumor drug for infusion to be contained in the liquid form drug packing unit 411, examples thereof include: an alkylating agent such as lomustine and melphalan; an antimetabolite such as gemcitabine hydrochloride; anti-cancer antibiotic preparations such as idarubicin hydrochloride, pirarubicine hydrochloride and epirubicin hydrochloride; anti-tumor vegetable component preparations such as irinotecan hydrochloride, paclitaxel, docetaxel, vinorelbine tartrate, nogitecan hydrochloride and etoposide; and cisplatin, neoplatin, carboplatin, mitoxantrone hydrochloride and rituximab. Moreover, from the viewpoint of liquid form drug stability, a glass vial is sometimes used as the liquid form drug container 41. Furthermore, with respect to the liquid form drug container 41, a barrel-type container that is made from glass or plastic, and has a movable rubber member and a gasket attached to the lower portion thereof, in the same manner as a syringe, may be used.

Here, the liquid form drug container 41 may be designed so that it is bag-filled with the approved volume or 5 to 10 times the approved volume of an anti-tumor drug for infusion. With this arrangement, mixed solutions can be adjusted and prepared for a plurality of patients, without the necessity of exchanging the liquid form drug containers 41. Moreover, a liquid form drug the does of which corresponds to a plurality of patients having an average body surface area is primary-diluted and adjusted preliminarily to form a bag product with a total liquid amount in a range of 50 to 1,000 mL. By using the liquid form drug container 41 filled with the primary-diluted liquid form drug, it is possible to control the does of medication more precisely. In this case, the port portion 41a of the liquid form drug container 41 is preferably provided with a dedicated connecting system to the mixing tube 3 for the diluent container 42 and the mixing container 43 so as to avoid misconception with other common containers or incorrect medication. In this case, in order to positively carry out the connection, the port portion may have a lure-type or clip-type securing system.

Moreover, in the case when a liquid form drug is poor in stability after having been dissolved or when a liquid form drug is unstable in a low concentration, as shown in Fig. 6(b), the liquid form drug container 41 may be prepared as a composite-chamber container made of a plastic bag that has a liquid form drug packing unit 411 filled with an anti-tumor drug serving as a liquid form drug A, a primary diluent packing unit 412 packing a primary diluent B used for primary-diluting the liquid form drug A, an easy-peeling seal 413 that serves as a communication unit which is placed between the liquid form drug packing unit 411 and the primary diluent packing unit 412 so as to mix the liquid form drug and the primary dilution solution, a suspending-device insertion hole 41b used for suspending the liquid form drug container 41 onto a suspending device such as a hook, a port portion 41a of the liquid form drug container 41 which is placed at a position opposite to the position at which the suspending-device insertion hole 41b is placed, and allowed to connect to the primary diluent packing unit 412, and connected to a liquid form drug container connecting port 31a of the mixing tube 3. The port portion 41a of the liquid form drug container 41 is provided with a liquid form drug closing portion 41c that seals the primary diluent packing unit 412. This liquid form drug closing portion 41c is designed in such a manner, when connected to the liquid form drug container connecting port 31a, the liquid form drug closing portion 41c is ruptured so as to allow the liquid form drug channel 31 of the mixing tube 3 to connect to the primary liquid form drug packing unit 412 of the liquid form drug container 41.

Here, the present embodiment has exemplified an arrangement in which the packing unit on the side that connects to the port portion 41a of the liquid form drug container 41; however, the packing unit on the side that attaches to the liquid form drug container connecting port portion 41 may be used as the liquid form drug packing unit. Here, the number of the packing units of the liquid form drug container is not limited to two, and at least two or more packing units are prepared. With this arrangement, one or more kinds of liquid form drugs and one or more kinds of diluents can be contained.

Moreover, as shown in Fig. 6(c), the liquid form drug container 41 may be prepared as a plastic bag that has a liquid form drug packing unit 411 filled with an anti-tumor drug serving as a liquid form drug A, a port portion 41a of the liquid form drug container 41 which allows the liquid form drug container 41 to plug into the liquid form drug packing unit 411, and is connected to the liquid form drug container connecting port 31a of the mixing tube 3, and a connection unit 41d that is placed at a position virtually opposite to the position at which the port portion 41a of the liquid form drug container 41, and allows connection to another liquid form drug container packing a primary diluent B used for primary-diluting the liquid form drug A so as to connect to the liquid form drug packing unit 411. The port portion 41a of the liquid form drug container 41 is provided with a liquid form drug closing portion 41c that seals the liquid form drug packing unit 411. This liquid form drug closing portion 41c is designed in such a manner, when connected to the liquid form drug container connecting port 31a, the liquid form drug closing portion 41c is ruptured so as to allow the liquid form drug channel 31 of the mixing tube 3 to connect to the liquid form drug packing unit 411 of the liquid form drug container 41. The connection unit 41d is provided with a liquid form drug closing portion 41f that tightly seals the liquid form drug packing unit 411, and a double-sided needle 41e that breaks the respective liquid form drug closing portions of the respective infusion solution containers upon connection between the liquid form drug container and the other liquid form drug container.

Here, this arrangement makes it possible to connect two or more liquid form drug containers so that an undiluted liquid form drug is contained in one of the liquid form drug containers, while a primary diluent being contained in another liquid form drug container, with these two containers being connected through a connecting unit; thus, a primary diluting process for the liquid form drug is available. Moreover, when three or more liquid form drug containers are connected, it becomes possible to carry out a sequence of diluting process than a secondary diluting process or the like.

(Diluent container)

In the embodiment of the present invention, the liquid form drug container 42 made of a plastic bag filled with 100 to 1,000 mL of saline or a 5 % glucose solution serving as the diluent, is used. The container port portion 42a of the diluent container is made of, for example, a medical rubber stopper.

(Mixing container)

In the embodiment of the present invention, a plastic mixing container 43 made from, for example, polyethylene, polypropylene, which is suitable for a pharmaceutical container, is used. With respect to the mixing container 43, a conventionally-used empty container that can be connected to the mixing tube 3 may be used, or an exclusively-used container may be utilized. Here, at the time of intact, the container is preferably united with the mixing tube 3 so as to maintain an aseptic state.

(Connection between mixing tube and liquid form drug container)

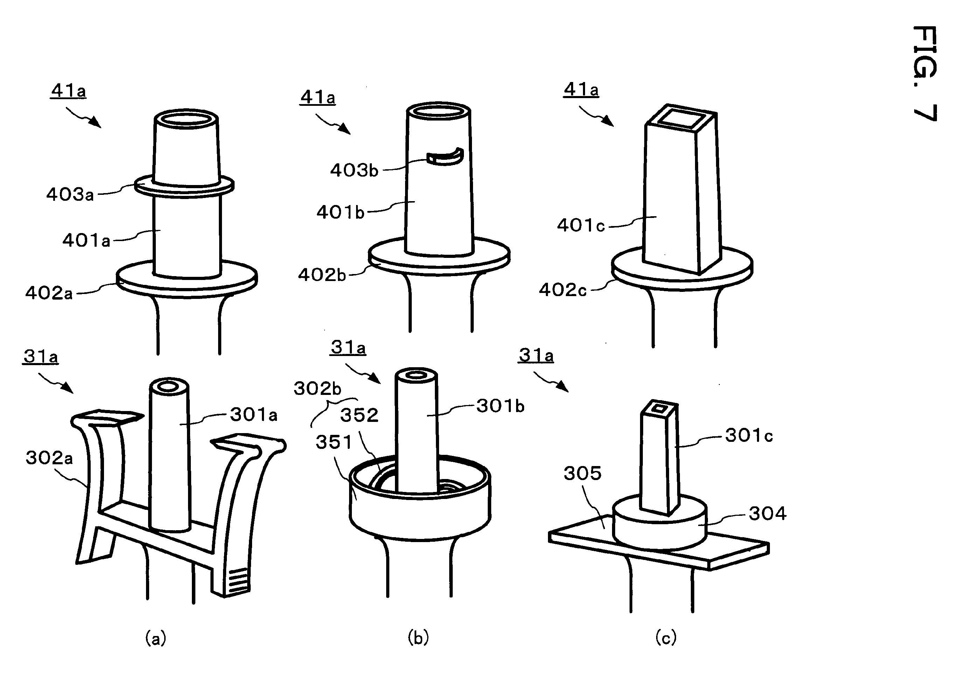

Fig. 7 is an outside drawing that shows an example of a combination between a liquid form drug container connecting port 31a of the mixing tube 3 and a port portion 41a of the liquid form drug container 41. The liquid form drug container connecting port 31a, which can be connected to the liquid form drug container 41, is formed into a shape that is not connectable to another container such as the diluent container 42.

First, the following description will discuss the mixing tube 3 and the liquid form drug container 41 of Fig. 7 (a). The liquid form drug container connecting port 31a of the mixing tube 3, shown in Fig. 7(a), is provided with a tube portion 301a having a cylinder shape, which attaches to the liquid form drug channel 31, and a clip 302a serving as a securing member for fixedly securing the port portion 41a of the liquid form drug container 41. Moreover, the port portion 41a of the liquid form drug container 41, shown in Fig. 7(a), is provided with a flange portion 402a formed into a disc shape, a tube portion 401a which is placed on the flange portion 402a, and has a cylinder-shaped inner circumferential face, to which the tube portion 301a of the liquid form drug container connecting port 31a is inserted, and a flange portion 403a to be secured, which has a disc shape and is placed on an outer circumferential face of the tube portion 401a so as to serve as a secured portion that is fixedly secured by the clip 302a of the liquid form drug container connecting port 31a. An elastic sealing member 41g, which can be pierced by the tube portion 301a in a manner so as to prevent external leakage of the liquid form drug, is placed inside the tube shaped portion 401a of the liquid form drug container 41. A rubber stopper or the like is utilized as the elastic sealing member 41g, and a structure that makes the liquid form drug container connecting port 31a easily pierced, that is, for example, a notch 41h, may be formed therein. The flange portion 403a is fixedly secured onto the clip 302a of the mixing tube 3. When the port portion 41a of the liquid form drug container 41 is connected to the liquid form drug container connecting port 31a of the mixing tube 3, the liquid form drug closing portion 41c, placed on the inner circumferential side of the tube portion 401a of the port portion 41a, is ruptured by the tube portion 301a of the liquid form drug container connecting port 31a so as to allow the liquid form drug channel 31 of the mixing tube 3 to combine with the liquid form drug packing unit 411 of the liquid form drug container 41.

Next, the following description will discuss the mixing tube 3 and the liquid form drug container 41 shown in Fig. 7(b). The liquid form drug container connecting port 31a of the mixing tube 3, shown in Fig. 7(b), is provided with a tube portion 301b having a cylinder shape, which combines with the liquid form drug channel 31, and a spiral-shaped securing portion 302b that serves as a securing member for fixedly securing the port portion 41a of the liquid form drug container 41. The spiral securing portion 302b has a spiral-shaped guide portion 352 formed into a spiral shape on the inner circumference of the cylinder portion 351. Moreover, the port portion 41a of the liquid form drug container 41, shown in Fig. 7(b), is provided with a flange portion 402b formed into a disc shape, a tube portion 401b which is placed on the flange portion 402b, and has a cylinder-shaped inner circumferential face, to which the tube portion 301b of the liquid form drug container connecting port 31a is inserted, and a protruding portion 403b to be secured, which has a protruding shape that is placed on an outer circumferential face of the tube portion 401b, and forms one portion of the spiral as the portion to be secured.

An elastic sealing member 41g, which can be pierced by the tube portion 301a in a manner so as to prevent external leakage of the liquid form drug, is placed inside the tube portion 401b of the liquid form drug container 41. A rubber stopper or the like is utilized as the elastic sealing member 41g, and a structure that makes the piercing process easier, that is, for example, a notch 41h, may be formed therein. With respect to the port portion 41a of the liquid form drug container 41 and the liquid form drug container connecting port 31a of the mixing tube 3, the protruding portion 403b to be secured of the liquid form drug container 41 guides the port portion 41a of the liquid form drug container 41 by using the spiral-shaped guide portion 352 so as to be secured. When the port portion 41a of the liquid form drug container 41 is connected to the liquid form drug container connecting port 31a of the mixing tube 3, the liquid form drug closing portion 41c, placed on the inner circumferential side of the tube portion 401b of the port portion 41a, is ruptured by the tube portion 301b of the liquid form drug container connecting port 31a so as to allow the liquid form drug channel 31 of the mixing tube 3 to combine with the liquid form drug packing unit 411 of the liquid form drug container 41.

The following description will discuss the mixing tube 3 and the liquid form drug container 41 relating to an example shown in Fig. 7(c). The liquid form drug container connecting port 31a of the mixing tube 3, shown in Fig. 7 (c), is provided with a flange portion 305 having a rectangular plate shape, a cylinder-shaped base portion 304 placed on the flange portion 305, and a tube portion 301c that is placed on a base portion 304 and communicates with the liquid form drug channel 31 having a square shape in its cross section. Here, the port portion 41a of the liquid form drug container 41, shown in Fig. 7(c), is provided with a flange portion 402c formed into a flange shape and a tube portion 401c which is placed on the flange portion 402c, and has an inner circumferential face having a square shape in its cross section, to which the tube portion 301c of the liquid form drug container connecting port 31a is inserted.

An elastic sealing member 41g, which can be pierced by the tube portion 301c in a manner so as to prevent external leakage of the liquid form drug, is placed inside the tube portion 401c of the liquid form drug container 41. A rubber stopper or the like is utilized as the elastic sealing member 41g, and a structure that makes the piercing process easier, that is, for example, a notch 41h, may be formed therein. When the port portion 41a of the liquid form drug container 41 is connected to the liquid form drug container connecting port 31a of the mixing tube 3, the liquid form drug closing portion 41c, placed on the inner circumferential side of the tube portion 401c of the port portion 41a, is ruptured by the tube portion 301c of the liquid form drug container connecting port 31a so as to allow the liquid form drug channel 31 of the mixing tube 3 to combine with the liquid form drug packing unit 411 of the liquid form drug container 41.

(Insertion of mixing tube)

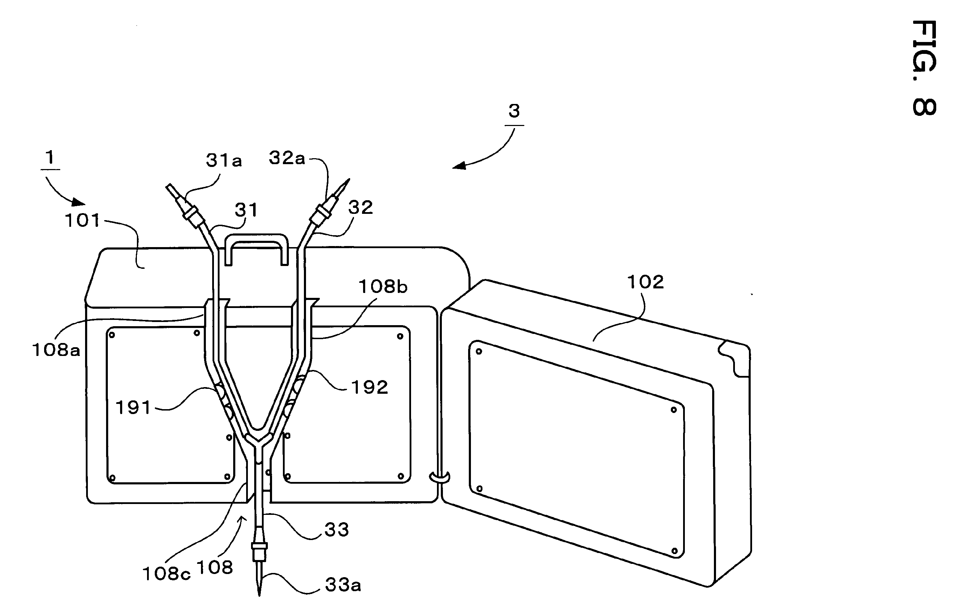

Fig. 8 shows an admixture preparation system in a state in which the mixing tube 3 is attached to the admixture machine 1. The mixing tube 3 is provided with a liquid form drug channel 31, a diluent channel 32 , and a mixing channel 33, and tip portions of the respective delivery units have a liquid form drug container connecting port 31a, a diluent container connecting port 32a and a mixing container connecting port 33a that are respectively connected to the liquid form drug container 41, the diluent container 42 and the mixing container 43. The liquid form drug channel 31, diluent channel 32, and mixing channel 33 of the mixing tube 3 are respectively attachable to a liquid form drug delivery unit guide 108a, a diluent delivery unit guide 108b and a mixture delivery unit guide 108c of the guide unit 108. A liquid form drug delivery unit 191 and a diluent delivery unit 192, placed at the liquid form drug delivery unit guide 108a and the diluent delivery unit guide 108b, are constituted by rollers, for example, and when the rollers are made in contact with the liquid form drug channel 31 and the diluent channel 32 attached to the liquid form drug delivery unit guide 108a and the diluent delivery unit guide 108b, and rotated, the liquid form drug and the diluent respectively contained in the liquid form drug channel 31 and the diluent channel 32 are individually delivered. In this embodiment, roller pumps in which the liquid delivery units are made of rollers have been exemplified; however, a peristaltic movement pump in which a plurality of pistons are placed so that the pistons are alternately moved may be used, and various structures in which, for example, contact is made onto a tube-shaped liquid delivery unit so that the liquid is delivered may be adopted.

Moreover, for example, a mixing tube insertion sensor may be placed in each of the liquid form drug delivery unit 19a and the diluent delivery unit 19b, and when the insertion of the mixing tube 3 into the guide unit 108 is insufficient, the warning unit 17 is allowed to give warning. The mixing tube insertion sensor may be placed in the vicinity of the delivery unit of the guide unit 108, and designed so that, upon contact with the mixing tube 3 or by using a photoelectric tube, it is allowed to detect whether or not the insertion of the mixing tube 3 into the guide unit 108 is insufficient, or may be allowed to detect the driving resistance of the delivery unit, that is, for example, the amount of the driven delivery unit (amount of rotation, etc.) in response to a driving signal inputted to the delivery unit, and designed so that, by comparing the driving resistance with a predetermined value, it is allowed to detect whether or not the insertion of the mixing tube 3 into the guide unit 108 is insufficient.

The present embodiment has exemplified a case in which the mixing tube to which the mixing tube 2 is inserted, which is formed as a separate member from the admixture machine 1, is inserted into the guide unit 108; however, a admixture preparation system in which a mixing tube to be inserted into the guide unit of the admixture machine is united with the admixture machine may be adopted.

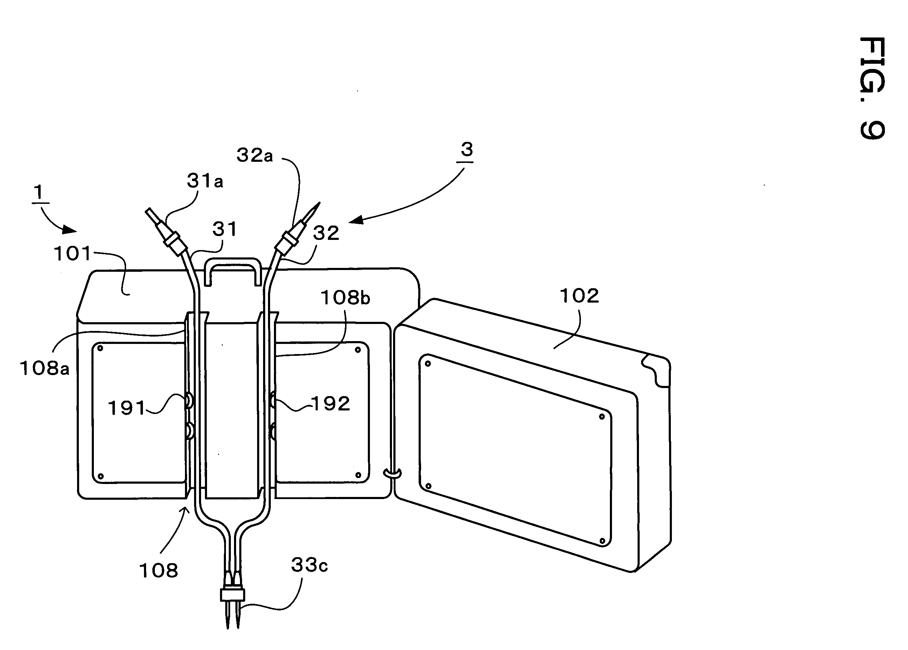

Fig. 9 shows an example of an admixture preparation system constituted by an admixture machine and a mixing tube relating to another embodiment of the present invention. In this example, the mixing tube 3 is independently placed without intersecting the liquid form drug channel 31 and the diluent channel 32, and integrally formed in mixing container connecting ports 33c. In the example of Fig. 9, two mixing container connecting ports 33c are formed in parallel with each other; however, these connecting ports may be formed into one portion so that the liquid form drug channel 31 and the diluent channel 32 are joined to each other at this portion. Moreover, in the admixture machine 1 shown in Fig. 9, the shape of the guide unit 108 is formed so that the liquid form drug channel guide 108a and the diluent channel guide 108b are arranged virtually in parallel with each other. In the guide unit 108 of this shape also, the above-mentioned mixing tube 3 shown in Fig. 5 can be utilized.

(Operation of admixture preparation system)

The following description will discuss the operation of an admixture preparation system in accordance with the embodiment of the present invention. First, a mixing tube 3, branched into a tong shape, is inserted into the guide unit 108 formed into a tong shape, with the door portion 102 of the admixture machine 1 being opened, and the door portion 102 is closed. When, upon closure of the door portion 102, the mixing tube insertion sensor detects that the insertion of the mixing tube 3 into the guide unit 108 is insufficient, the control unit 11 controls the warning unit 17 to give warning. The liquid form drug container 41, the diluent container 42 and the mixing container 43 are respectively connected to the tips of the liquid form drug channel 31, the diluent channel 32 and the mixing channel 33 of the mixing tube 3. Moreover, items, such as the name of a patient for medication, the body surface area of the patient for medication, the name of a liquid form drug, the does of the liquid form drug, the name of a diluent, the amount of the diluent, the date of admixture and the person in charge of admixture, are inputted to the information input unit 13 through the operation unit 13a. The calculation unit 14 automatically calculates the value of the does of the liquid form drug based upon the body surface area of the patient for medication inputted into the information input unit 13. The control unit 11 compares the does of the liquid form drug individually supplied from the information input unit 13 with the calculated value of the does of the liquid form drug automatically calculated by the calculation unit 14, and when the difference is greater than a predetermined value, controls the warning unit 17 to give warning. Moreover, based upon names of the diluent that are not applicable, and recorded in the memory unit 15 in association with the specific liquid form drug, the control unit 11 determines whether or not the diluent the name of which is individually inputted from the information input unit 13 is inapplicable to the liquid form drug the name of which is inputted, and when it determines that the diluent the name of which is individually inputted from the information input unit 13 is inapplicable to the liquid form drug the name of which is inputted, controls the warning unit 17 to give warning.

In accordance with the information inputted from the information input unit 13, the calculation unit 14 determines the does of the liquid form drug and the amount of the diluent to be supplied based upon the information supplied from the information input unit 13, and based upon the amounts, calculates the respective amounts of delivery per unit time of the liquid form drug and the diluent. The control unit 11 compares the does of the liquid form drug individually supplied from the information input unit 13 with the calculated value of the does of the liquid form drug automatically calculated by the calculation unit 14, and when the difference is smaller than the predetermined value, drives the liquid form drug delivery unit 19a and the diluent delivery unit 19b based upon the respective amounts of liquid delivery per unit time of the liquid form drug and the diluent, calculated in the calculation unit 14. Thus, a mixed solution consisting of the does of the liquid form drug and the amount of the diluent to be supplied is contained in the mixing container 43. When the mixed solution has been contained in the mixing container 43, the control unit 11 drives the printing device 6 through the interface unit 16 so as to print at least one portion of pieces of information inputted to the information input unit 13, such as the name of a patient for medication, the body surface area of the patient for medication, a liquid form drug name, the does of the liquid form drug, a diluent name, the amount of the diluent, the date and time of admixture and the person in charge of the admixture, on a pasting label that is attached to the mixing container 43. The printing items, prepared by the printing device 6, may be prepared as characters indicating items, such as the name of a patient for medication, the body surface area of the patient for medication, a liquid form drug name, the does of the liquid form drug, a diluent name, the amount of the diluent, the date and time of admixture and the person in charge of the admixture, or may be prepared as various pieces of information, such as bar codes, indicating these pieces of information.

The pasting label having such printed items is affixed to the mixing container 43 so that the information required for medication of the mixed solution is recognized. Moreover, upon preparation a plurality of mixed solutions, the mixing container is exchanged so that the next mixing container is connected to the mixing tube, and next items, such as the name of the next patient for medication, the body surface area of the patient for medication, the name of a liquid form drug, the does of the liquid form drug, the name of a diluent, the amount of the diluent, the date of admixture and the person in charge of admixture, are inputted to the information input unit 13 through the operation unit 13a. Thereafter, the same operation is repeated in the admixture preparation system.

Although the above-mentioned embodiment has exemplified a case in which the body surface area of a patient for medication is inputted to the information input unit 11 through the operation unit 13a, another system may be used in which, based upon a patient name for medication inputted through the operation unit 13a, the body surface area of the patient, recorded in the memory unit 15 in association with the name of the patient, is read and inputted to the information input unit 11; thus, the inputting operation of the body surface area of the patient for medication through the operation unit 13a may be omitted. With respect to the name of the patient, not only text data indicating the name, but also number, alphabets, symbol or ID codes consisting of the combination of these data may be used. Moreover, the patient name may be read through bar codes attached to the name plate of the patient or the bed or the like of the patient, by using a bar code reader 133, and inputted to the information input unit 11. Similarly, a signal is transmitted from an IC tag exclusively-used by the patient, and the signal may be received and inputted. Furthermore, a memory card on which a magnetic tape or the like recording patient name is stuck is detachably attached to patient's clothes or the patient's bed as a name tag, and by reading the memory card by a card reader 136, the data can be inputted to the information input unit 11. With this arrangement, the inputting process of patient names to the information input unit 11 can be simplified, and an erroneous inputting process of a patient name can be prevented. Here, an IC card using an integrated circuit may be adopted as the memory card.

Moreover, with respect to the name of a liquid form drug and the name of a diluent, not only text data indicating the names thereof, but also numbers, alphabets, symbols or ID codes consisting of the combination of these data may be used. Although the above-mentioned embodiment has exemplified a case in which the name of a liquid form drug and the name of a diluent are inputted to the information input unit 11 through the operation unit 13a, another system may be used in which bar codes attached to the liquid form drug container, the diluent container and the like are read by using a bar code reader 133 so that the read data may be inputted to the information input unit 11. Similarly, a memory card on which a magnetic tape or the like recording a liquid form drug name and a diluent name is stuck is tied to the liquid form drug container and the diluent container by using a string or the like, and by reading the memory card by a card reader 136, the data can be inputted to the information input unit 11. With this arrangement, the inputting process of the liquid form drug name and the diluent name to the inf ormation input unit 11 can be simplified, and an erroneous inputting process of the liquid form drug name and the diluent name can be prevented.

Here, with respect to the predetermined information to be inputted to the information input unit 13, data received from an ordering system, an electronic medical report system or the like through a communication device 7 may be inputted through on-line inputs or the like through the interface unit 16. In this case, for example, when a patient name is inputted to the operation unit 13b, the control unit 11 drives the communication device 7 through the interface unit 16 so that communications are made with the ordering system and the electronic medical report system to receive predetermined necessary information so that the information is inputted to the information input unit 13. With this arrangement, it is possible to share information with the ordering system, the electronic medical report system and the like.

Although the present embodiment has exemplified a case in which the information required for medication of the mixed solution in the mixing container is recognized through the attached label, another arrangement may be used in which an IC tag is attached to the mixing container 43 so that the data is recognized. In this case, the control unit 11 drives a radio terminal 7 through the interface unit 16 so that communication is made with the IC tag attached to the mixing container to write required information for medication in the IC tag.

(Removal of the mixing container)

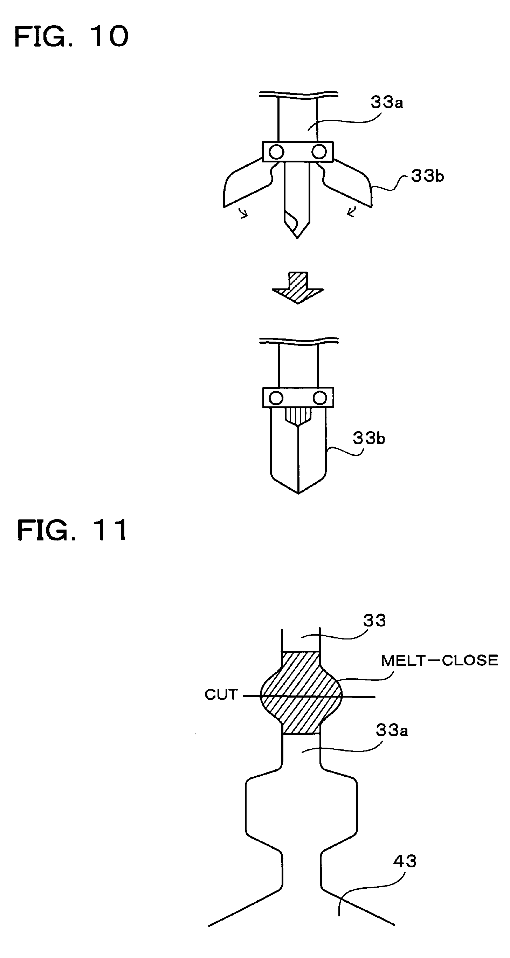

Upon completion of a series of admixing and preparation operations, the mixing container is removed from the mixing tube. Here, in the case when the mixing container and the mixing tube are not formed into an integral part, the tube is simply pulled out, and a sealing member such as a cap is attached to the port of the mixing container, if necessary, so that the container is sealed. In this case, a cover part 33b, for example, shown in Fig. 10, is preferably placed on the tip of the mixing tube so as to prevent residual liquid form drug from dripping.

In the case when the mixing container and the mixing tube are formed into an integral part, for example, as shown in Fig. 11, the mixing tube is melted to be closed near the connecting port 33a, and cut at this portion. With respect to such a melt-closing and cutting means, conventionally known means and means to be developed in the future may be used, and these means may be attached to the admixture machine in accordance with the embodiment of the present invention.

INDUSTRIAL APPLICABILITY

As described above, the admixture machine, admixture preparation system, mixing tube, liquid form drug container, mixing container and admixture method of infusion in accordance with the present invention are effectively used for diluting and admixing, for example, an anti-cancer drug and the like.

**Claims**

1.  A admixture machine for infusion, which is used for admixture preparation predetermined does of a liquid form drug to be administrated to a patient and a diluent, comprising:

    an information input unit for inputting predetermined information involving data concerning at least the body surface area, the area under the plasma drug concentration curve (AUC) or the body weight of the patient;
    a liquid delivery amount calculation unit for determining the does of a liquid form drug and the amount of a diluent which are both to be delivered based upon the information inputted through the information input unit and calculating the delivery does of the liquid form drug and the diluent based upon the determined amounts thereof;
    a guide unit for inserting a mixing tube which provides channels of the liquid form drug and the diluent; and liquid delivery units which are in contact respectively with a liquid form drug channel through which the liquid form drug flows and a diluent channel through which the diluent flows in the mixing tube to be inserted into the guide unit and thus deliver the liquid form drug and the diluent based on the delivery does of the liquid form drug and the diluent as determined in the liquid delivery amount calculation unit.

2.  The admixture machine for infusion according to claim 1, wherein the predetermined information to be inputted to the information input unit further comprises a name of a patient for medication; the admixture machine for infusion further comprises a memory unit capable of recording at least the name of the patient, the body surface area, the AUC or the body weight of the patient in association with one another; and upon input of a name of a patient into the information input unit, at least the body surface area, the AUC or the body weight in association with the inputted patient name is called for by reference to the memory unit, and automatically inputted to the information input unit.

3.  The admixture machine for infusion according to claim 1 or 2, wherein the predetermined information to be inputted to the information input unit further comprises the body surface area of the patient for medication, the AUC, the body weight, the name of a liquid form drug to be administrated, the does of the liquid form drug, and the does of the liquid form drug per unit body surface area or per body weight of the patient with respect to the liquid form drug or the does of the liquid form drug determined based upon the AUC, and the calculation unit automatically calculates the value of the does of the liquid form drug based upon at least the body surface area, the AUC or the body weight of the patient inputted to the information input unit, and the admixture machine for infusion further comprises a memory unit that is capable of recording the name of the liquid form drug and the does of the liquid form drug per unit body surface area or per weight or the does of the liquid form drug determined based upon the AUC in association with one another and a warning unit which compares the does of the liquid form drug individually inputted from the information input unit with the value of the does of the liquid form drug automatically calculated in the calculation unit, and, when the difference is greater than a predetermined value, gives warning.

4.  The admixture machine for infusion according to claim 3, wherein the memory unit further records at least one piece of information selected from application and medication methods.

5.  The admixture machine for infusion according to claim 1 or 2, wherein based upon information given from the information input unit, the calculation unit automatically calculates the does of the liquid form drug and the amount of the diluent to be supplied.

6.  The admixture machine for infusion according to any of claims 1 to 5, wherein with respect to the inputting process of the body surface area or the AUC of the patient, by inputting parameters required for calculating the body surface area or the AUC, calculations are automatically carried out through predetermined operation expressions based upon these parameters so that the results are automatically inputted.

7. The admixture machine for infusion according to any of claims 1 to 6, further comprising:

    a bar-code reading unit which is capable of inputting at least one portion of pieces of information to be inputted to the information input unit by reading bar codes.

8. The admixture machine for infusion according to any of claims 1 to 6, further comprising:

    a signal receiving unit which is capable of inputting at least one portion of pieces of information to be inputted to the information input unit by using IC tag signals.

9. The admixture machine for infusion according to any of claims 1 to 8, further comprising:

    a memory-card reading unit which is capable of inputting at least one portion of pieces of information to be inputted to the information input unit by reading a memory card.

10. The admixture machine for infusion according to any of claims 1 to 9, wherein a printing device, which is capable of printing at least one portion of pieces of information to be inputted to the information input unit on a pasting label, is prepared in a connectable state.

11. The admixture machine for infusion according to any of claims 1 to 10, further comprising:

    a flow meter for measuring the amount of flow passing through a mixing tube to be inserted to the guide unit,

    wherein the amount of flow, measured by the flow meter, is compared with the amount of liquid delivery calculated by the calculation unit so that the amount of liquid delivery is controlled through a feed-back controlling process.

12. The admixture machine for infusion according to any of claims 1 to 11, wherein the liquid form drug is an anti-tumor drug for infusion.

13. The admixture machine for infusion according to any of claims 1 to 12, wherein the mixing tube to be inserted to the guide unit of the admixture machine for infusion is branched into a tong shape, that is, a tong structure having one of branched ends serving as a liquid form drug channel to be connected to a liquid form drug container for packing a liquid form drug, the other end serving as a diluent channel to be connected to a diluent container for packing a diluent, and the branch portion serving as a mixing channel in which the liquid form drug channel and the diluent channel are joined to each other, and the guide unit of the admixture machine for infusion is formed into a tong shape to which the tong-shaped mixing tube is inserted.

14. A mixing tube, which is the mixing tube to be used in the admixture machine for infusion according to any of claims 1 to 13, having:

    a structure branched into a tong shape, that is, a tong structure having one of branched ends serving as a liquid form drug channel to be connected to a liquid form drug container for packing a liquid form drug, the other end serving as a diluent channel to be connected to a diluent container for packing a diluent, and the branch portion, in which the liquid form drug channel and the diluent channel are joined to each other, serving as a mixing channel to be connected to a mixing container for packing the mixed solution of the liquid form drug and the diluent.

15. The mixing tube, which is the mixing tube to be used in the admixture machine for infusion according to any of claims 1 to 13, wherein the mixing tube comprises a liquid form drug channel one end of which is connected to a liquid form drug container for packing a liquid form drug and a diluent channel one end of which is connected to a diluent container for packing a diluent individually, with the other ends of the channels being connected to a mixing container for packing a mixed solution of the liquid form drug and the diluent.

16. The mixing tube according to claim 14 or claim 15, wherein the mixing tube comprises a liquid form drug container connecting port to be connected to a port of the liquid form drug container at an end of the liquid form drug channel and a diluent container connecting port to be connected to a port of the diluent container at an end of the diluent channel, with the liquid form drug container connecting port being formed into a shape that is only connectable to the liquid form drug container.

17. The mixing tube according to any of claims 14 to 16, wherein the liquid form drug container connecting port of the mixing tube comprises a securing member used for securing the port of the liquid form drug container.

18. A liquid form drug container, which is the liquid form drug container to be used in the admixture machine for infusion according to any of claims 1 to 13, comprising: a discharging outlet that is connected to a liquid form drug container connecting port placed at an end portion of the liquid form drug channel, and is placed at the port of the liquid form drug container.

19. The liquid form drug container according to claim 18, wherein liquid form drugs that have been primarily diluted are contained in the liquid form drug container in a plurality of amounts to be applied.

20. The liquid form drug container according to claim 18, wherein undiluted liquid form drugs are contained in the liquid form drug container in a plurality of amounts to be applied.

21. The liquid form drug container according to claim 18, comprising:

   a liquid form drug packing unit for packing the liquid form drug, and
   a primary diluent packing unit for packing a primary diluent that is used for primarily diluting the liquid form drug,

   wherein a communicating unit, which is used for mixing the liquid form drug and the primary diluent, is placed between the liquid form drug packing unit and the primary diluent packing unit.

22. The liquid form drug container according to claim 21, wherein the liquid form drug container is a plastic bag having two chambers separated by an easily-peelable seal, with one of the chambers packing an undiluted liquid form drug or unreconstituted injectable drug and the other chamber packing a primary diluent.

23. The liquid form drug container according to claim 18, wherein the liquid form drug container comprises a connecting unit, placed at a position virtually opposing to the port of the liquid form drug container, which allows transfer with another liquid form drug container.

24. The liquid form drug container according to claim 23, wherein the connecting unit is provided with a double-sided needle.

25. A mixing container, which is used in the admixture machine for infusion according to claims 1 to 13, wherein the mixing container is integrally formed with the mixing tube.

26. A admixture preparation system comprising the admixture machine for infusion and the mixing tube according to any of claims 1 to 13,
   wherein the mixing tube to be inserted to the guide unit of the admixture machine for infusion has a structure branched into a tong shape, that is, a tong structure having one of branched ends serving as a liquid form drug channel to be connected to a liquid form drug container for packing a liquid form drug, the other end serving as a diluent channel to be connected to a diluent container for packing a diluent, and the branch portion, in which the liquid form drug channel and the diluent channel are joined to each other, serving as a mixing channel to be connected to a mixing container for packing the mixed solution of the liquid form drug and the diluent; and the guide unit of the admixture machine for infusion comprises at least a liquid form drug channel insertion unit to which the liquid form drug channel is inserted and a diluent channel insertion unit to which the diluent channel is inserted.

27. A admixture preparation system of infusion comprising the admixture machine for infusion and the mixing tube according to any of claims 1 to 13,
   wherein the mixing tube to be inserted to the guide unit of the admixture machine for infusion comprises a liquid form drug channel that is connected to a liquid form drug container for packing a liquid form drug and a diluent channel that is connected to a diluent container for packing a diluent, with one end of the liquid form drug channel being connected to the liquid form drug container and one end of the diluent channel being connected to the diluent container; and the guide unit of the admixture machine for infusion comprises at least a liquid form drug channel insertion unit to which the liquid form drug channel is inserted and a diluent channel insertion unit to which the diluent channel is inserted.

28. The admixture preparation system of infusion according to claim 26 or claim 27, wherein the mixing tube to be inserted to the guide unit of the admixture machine for infusion is integrally formed with the admixture machine for infusion.

29. The admixture preparation system of infusion according to claim 26 or claim 27, further comprising:

a container weight-measuring unit which allows controls on the does of the liquid form drug and the diluent by feeding back weight changes in the diluent container or the mixing container.

30. A admixture method of infusion in which a admixture machine for admixture preparation predetermined does of a liquid form drug to be administrated to a patient and a diluent is used, comprising the steps of:

inputting predetermined information involving data concerning at least the body surface area, the area under the plasma drug concentration curve (AUC) or the body weight of the patient to the admixture machine for infusion;
allowing the admixture machine for infusion to determine the does of a liquid form drug and the amount of a diluent which are both to be delivered based upon the inputted information, and also to calculate the delivery does of the liquid form drug and the diluent based upon the determined amounts thereof; and
based on the delivery does of the liquid form drug and the diluent that have been calculated, allowing liquid delivery units, formed at a guide unit for inserting a mixing tube which provides channels of the liquid form drug and the diluent to the admixture machine for infusion, to respectively contact with the liquid form drug channel forming a channel for the liquid form drug and the diluent channel forming a channel for the diluent in the mixing tube so that the liquid form drug and the diluent are respectively delivered.

# FIG. 1

(a)

| | |
|---|---|
| MODE SWITCHING | |
| ITEM SWITCHING | |
| ENTER | |
| CONFIRM／EXECUTE | |
| CANCEL／RESET | |
| STOP | |

| 1 | 2 | 3 |
|---|---|---|
| 4 | 5 | 6 |
| 7 | 8 | 9 |
| 10 | · | * |

(b)

FIG. 2

EP 1 563 819 A1

| PRINTING DEVICE 6 | COMMUNICATION DEVICE 7 | RADIO TERMINAL 8 |
|---|---|---|

| MEMORY UNIT 15 | I/F UNIT 16 | WARNING UNIT 17 |
|---|---|---|

| | CONTROL UNIT 11 | POWER-SUPPLY MONITORING UNIT 18 |
|---|---|---|

| DISPLAY UNIT 12 | INFORMATION INPUT UNIT 13 | CALCULATION UNIT 14 |
|---|---|---|

| OPERATION UNIT 13a | INFORMATION READING UNIT 13b |
|---|---|

41
42
41a
42a
31a
32a
31
32
19a
19
19b
33
43a
33a
43

# FIG. 3

D   K   S

```
        ┌─────────────┐
    S──→│  MULTIPLIER │  V
    K──→│     141     │──┐
        └─────────────┘  │   ┌──────────────┐
                         └──→│  COMPARATOR  │──→ C
    D───────────────────────→│     142      │
                             └──────────────┘
```

# FIG. 4

RADIO TERMINAL 8

WARNING UNIT 17

POWER-SUPPLY MONITORING UNIT 18

CALCULATION UNIT 14

COMMUNICATION DEVICE 7

I/F UNIT 16

CONTROL UNIT 11

INFORMATION INPUT UNIT 13

INFORMATION READING UNIT 13b

PRINTING DEVICE 6

MEMORY UNIT 15

DISPLAY UNIT 12

OPERATION UNIT 13a

42
44
42a
32a
32
33
33a
43a
41
41a
31a
31
43
44

# FIG. 5

# FIG. 6

(a)

(b)

(c)

# FIG. 7

(a)

(b)

(c)

# FIG. 8

## FIG. 9

## FIG. 10

33a

33b

33b

## FIG. 11

33

MELT—CLOSE

CUT

33a

43

<div align="center">

**INTERNATIONAL SEARCH REPORT**

</div>

| | International application No. |
|---|---|
| | PCT/JP03/14605 |

**A. CLASSIFICATION OF SUBJECT MATTER**
Int.Cl$^7$ A61J3/00, A61M5/14

According to International Patent Classification (IPC) or to both national classification and IPC

**B. FIELDS SEARCHED**

Minimum documentation searched (classification system followed by classification symbols)
Int.Cl$^7$ A61J3/00, A61M5/00-5/175

Documentation searched other than minimum documentation to the extent that such documents are included in the fields searched

| | | | |
|---|---|---|---|
| Jitsuyo Shinan Koho | 1926-1996 | Toroku Jitsuyo Shinan Koho | 1994-2004 |
| Kokai Jitsuyo Shinan Koho | 1971-2004 | Jitsuyo Shinan Toroku Koho | 1996-2004 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | WO 98/31322 A1 (TEEPLE, Edward, Jr.),<br>23 July, 1998 (23.07.98),<br>Full text; all drawings<br>& JP 2001-509059 A  & EP 671959 A1<br>& US 5925014 A | 1-11,30<br>12-29 |
| Y | US 5569181 A (Medrad, Inc.),<br>29 October, 1996 (29.10.96),<br>Full text; particularly, column 5, lines 6 to 22;<br>all drawings<br>& JP 09-503419 A  & WO 95-11722 A1<br>& EP 726789 A | 1-30 |
| Y | JP 61-29364 A (The University of Melbourne),<br>10 February, 1986 (10.02.86),<br>Full text; all drawings<br>& EP 164904 A2  & US 4741732 A | 1-30 |

| [x] Further documents are listed in the continuation of Box C. | [ ] See patent family annex. |
|---|---|

| | |
|---|---|
| *   Special categories of cited documents:<br>"A"  document defining the general state of the art which is not considered to be of particular relevance<br>"E"  earlier document but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search<br>12 February, 2004 (12.02.04) | Date of mailing of the international search report<br>02 March, 2004 (02.03.04) |
|---|---|
| Name and mailing address of the ISA/<br>Japanese Patent Office | Authorized officer |
| Facsimile No. | Telephone No. |

Form PCT/ISA/210 (second sheet) (July 1998)

**EP 1 563 819 A1**

### INTERNATIONAL SEARCH REPORT

International application No.

PCT/JP03/14605

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | US 6042564 A (Nstec S.A.),<br>28 March, 2000 (28.03.00),<br>Full text; all drawings<br>& WO 98/46293 A1      & EP 929331 A | 13-17<br>1-12,13-30 |

Form PCT/ISA/210 (continuation of second sheet) (July 1998)

31